# EUROPEAN PATENT APPLICATION

(11) **EP 2 522 487 A1**
(43) Date of publication of application: **14.11.2012**
(21) Application number: 11731853.5
(22) Date of filing: 07.01.2011
(51) Int. Cl.: B29C 55/18, A61F 13/15, A61F 13/49

(54) **DRAWING DEVICE AND METHOD OF MANUFACTURING ABSORPTIVE ARTICLE**

(30) Priority: 08.01.2010 JP 2010003426
(71) Applicant: Unicharm Corporation, Ehime 799-0111 (JP)
(72) Inventor: OKU, Tomomi, Kanonji-shi Kagawa 769-1602 (JP); SAKAGUCHI, Satoru, Kanonji-shi Kagawa 769-1602 (JP); OGASAWARA, Yoshikazu, Kanonji-shi Kagawa 769-1602 (JP); ITO, Noriaki, Kanonji-shi Kagawa 769-1602 (JP)
(74) Representative: Eke, Philippa Dianne
(86) International application number: PCT/JP2011/050202
(87) International publication number: WO 2011/083855

(57) **Abstract**

A manufacturing method for an absorptive article includes a sheet-stretching step S30 for performing a stretching process to stretch a continuous body of the exterior sheet 120 in a widthwise direction while conveying a continuous body. In the step of performing the stretching process, the continuous body is stretched by a stretching apparatus 500 including a first roll and a second roll. An axial core of the first roll and an axial core of the second roll correspond to the widthwise direction of the continuous body. The first roll and the second roll have surfaces each provided with columns of tooth units and untoothed units.

## Description

### [Technical Field]

The present invention relates to a stretching apparatus which performs a stretching process in a widthwise direction orthogonal to a conveyance direction of a continuous body, and to a method of manufacturing an absorbent article using the stretching apparatus.

### [Background Art]

Conventionally, an absorbent article such as a pants-type diaper, a sanitary napkin, or a panty liner, has been required to prevent leakage of bodily fluid of a wearer from the absorbent article even when the wearer moves. Thus, there has been known a technique in which a space (so-called pocket) for catching bodily fluid and excretions is formed between the crotch of the wearer and an absorber by facilitating the stretchability of the absorbent article in the widthwise direction thereof (for example, Patent Literature 1).

In order to form the aforementioned pocket, first, a stretching process is performed on the entire surface of a continuous body of an exterior sheet configuring the absorbent article. Subsequently, strand-shaped elastic members having elasticity are arranged in a stretched-out state on the both side edges of the absorber configuring the absorbent article.

The strand-shaped elastic member is naturally restored to a pre-stretch state, so that a region of the absorbent article, on which the strand-shaped elastic member has been arranged is contracted to form the pocket between the crotch of the wearer and the absorber. Accordingly, the bodily fluid and the excretions can be caught, thereby being able to prevent the leakage out of the absorbent article(so-called side leakage).

### [Citation List]

### [Patent Literature]

[PTL 1] Japanese Patent Unexamined Publication No. 2002-316359 (Page Nos. 2-3, Fig. 1)

### [Summary of Invention]

However, the aforementioned conventional manufacture of absorbent articles had the below-mentioned problem. That is, the stretched continuous body is thinner as compared to a case where it is not stretched. Therefore, in a case where the entire surface of the continuous body is stretched, the continuous body is more likely to have a turning up of its edge portions and formation of wrinkles, thereby causing a problem that the possibility of defects occurring is increased in a manufacturing process.

Therefore, an object of the present invention is to provide a stretching apparatus and a method of manufacturing an absorbent article using the stretching apparatus, the stretching apparatus being capable of reducing the occurrence possibility of defects occurring in a case where a continuous body of an exterior sheet or the like configuring the absorbent article is stretched in a manufacturing process of the absorbent article.

The present invention has therefore been made in consideration of the above-described problems inherent in the related art. It is an object of the present invention to provide a stretching apparatus configured to perform a stretching process for stretching a continuous body of a material in a widthwise direction orthogonal to a conveyance direction of the continuous body of the material, the stretching apparatus comprising:
a first roll and a second roll disposed to nip the continuous body of the material, wherein: an axial core of the first roll and an axial core of the second roll correspond to the widthwise direction of the continuous body of the material, and the first roll and the second roll have a width equal to at least a width of the continuous body of the material; the first roll and the second roll have surfaces, each provided with: an embossing region provided with a plurality of columns of tooth units and untoothed units, the tooth units being continuous in a circumferential direction of the first roll and the second roll, the untoothed units being formed between the tooth units; and a non-embossing region not provided with the tooth units and the untoothed units; the embossing region is formed on each of the surfaces, in a central region on an inner side in a widthwise direction of the first roll and the second roll; the non-embossing region is formed on the outer side in the widthwise direction of the first roll and the second roll with respect to the central region; and the continuous body of the material is conveyed in the conveyance direction while being sandwiched between the first roll and the second roll. [Brief Description of Drawings]

[Fig. 1] Fig. 1 is a plan view showing a disposable diaper 1 as an example of an absorbent article manufactured by a method of manufacturing an absorbent article according to a first embodiment.
[Fig. 2] Fig. 2 is a cross-sectional view of the disposable diaper 1.
[Fig. 3] Fig. 3 is a cross-sectional view of the disposable diaper 1.
[Fig. 4] Fig. 4(a) is a plan view showing an exterior sheet 20 of the disposable diaper 1, and Fig. 4(b) is an enlarged cross-sectional view (taken along the line A-A of Fig. 4(b)) showing the exterior sheet 20 of the disposable diaper 1.
[Fig. 5] Fig. 5 is a view for illustrating a method of manufacturing the absorbent article according to the first embodiment.
[Fig. 6] Fig. 6 is a perspective view showing a stretching apparatus 500 used in the method of manufacturing the absorbent article according to the first embodiment.
[Fig. 7] Fig. 7 is a front view of the stretching apparatus 500.
[Fig. 8] Fig. 8 is a side view of the stretching apparatus 500.
[Fig. 9] Fig. 9(a) is a front view of the stretching apparatus 500 according to the first embodiment, and Fig. 9(b) is an enlarged cross-sectional view showing an enlarged cross section of tooth units and untoothed units of the stretching apparatus 500.
[Fig. 10] Fig. 10 is a perspective view showing a stretching apparatus 500A used in a method of manufacturing an absorbent article according to a second embodiment.
[Fig. 11] Fig. 11 is a plan view showing an exterior sheet 20 of a disposable diaper 1A on which the stretching apparatus 500A performs a stretching process.

### [Description of Embodiments]

Hereinafter, a method of manufacturing an absorbent article and a stretching apparatus according to the present invention are explained with reference to drawings. Specifically, the first embodiment, the second embodiment, and other embodiments are explained.

In the following description of the drawings, the same or similar reference numerals are used to designate the same or similar parts. It will be appreciated that the drawings are schematically shown and the ratio and the like of each dimension are different from the real ones. Therefore, a specific dimension should be determined in view of the following description. Moreover, among the drawings, the respective dimensional relations or ratios may naturally differ.

### [First Embodiment]

First of all, a configuration of an absorbent article manufactured by a method of manufacturing an absorbent article according to the first embodiment is explained with reference to the drawings. In the present embodiment, the absorbent article means a tape-type disposable diaper. In the present embodiment, the tape- type diaper means a diaper that is in a spread-out state prior to use, rather than being formed in the shape of pants beforehand, and is worn by a wearer by fastening the predetermined portions of the product to each other with a tape and the like.

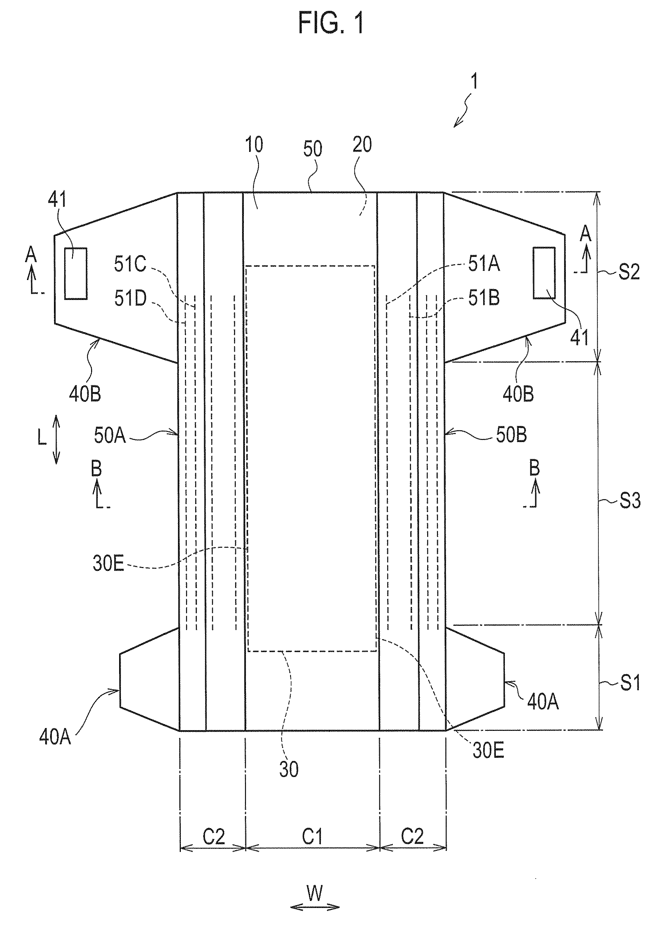

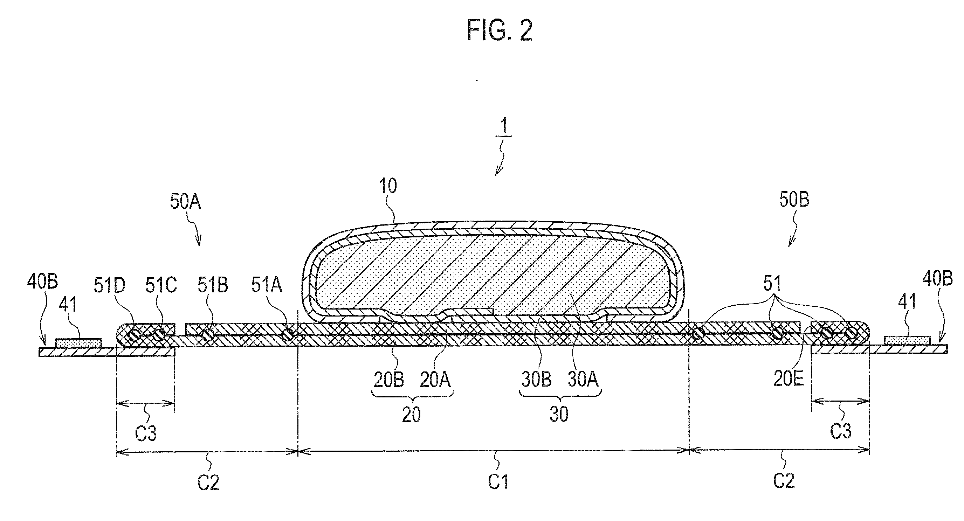

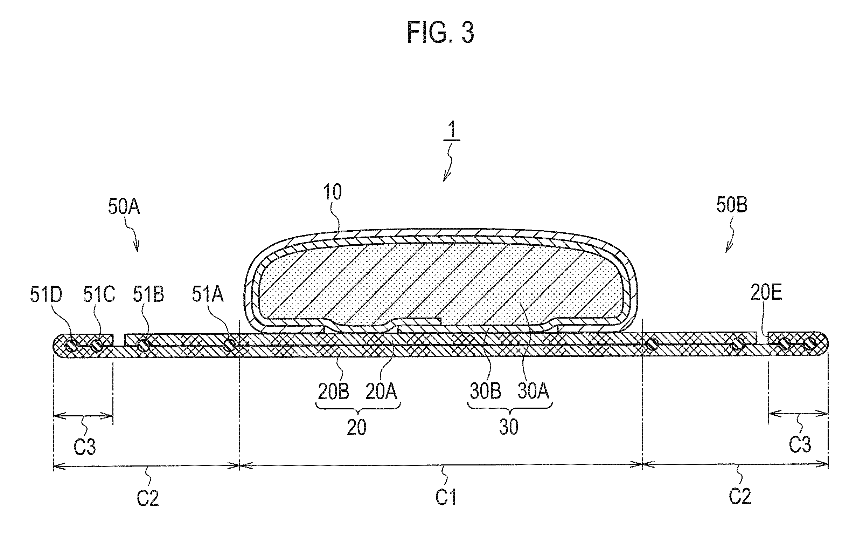

Fig. 1 is a plan view showing a disposable diaper 1. Fig. 2 is a cross-sectional view (taken along the line A-A of Fig. 1) showing the disposable diaper 1. Fig. 3 is a cross-sectional view (taken along the line B-B of Fig. 1) showing the disposable diaper 1.

As shown in Figs. 1 to 3, the disposable diaper 1 has a stretched shape in a front-back direction (hereinafter, referred to as a longitudinal direction L of the disposable diaper 1) corresponding to a direction extending from a front waistline (a ventral side) to a back waistline (a dorsal side) of a wearer.

In the longitudinal direction L of the disposable diaper 1, the disposable diaper 1 has a front waistline region S1 corresponding to the front waistline of the wearer, a back waistline region S2 corresponding to the back waistline of the wearer, and a crotch region S3 corresponding to the crotch of the wearer, positioned between the front waistline region S1 and the back waistline region S2.

Further, in a widthwise direction W orthogonal to the longitudinal direction L of the disposable diaper 1, the disposable diaper 1 has a central region C1 including an absorber 30 described later, and side regions C2 positioned outside the central region C1 with respect to the widthwise direction W of the disposable diaper 1.

The disposable diaper 1 described above includes a topsheet 10, an exterior sheet 20, and the absorber 30. Here, the topsheet 10, the exterior sheet 20, and the absorber 30 configure a main body 50. The main body 50 of the disposable diaper 1 has a rectangular shape. One end of the main body 50 is provided with a pair of ventral side flaps 40A. The other end of the main body 50 is provided with a pair of dorsal side flaps 40B.

The topsheet 10 is provided on the side that is in contact with the skin of the wearer. The topsheet 10 enwraps the absorber 30. The topsheet 10 is formed by a liquid-permeable sheet, such as a hydrophilic nonwoven cloth and woven cloth, an apertured plastic film, or an apertured hydrophobic nonwoven cloth.

As shown in Fig. 2, the exterior sheet 20 has a back nonwoven fabric 20B that is in contact with clothing, and a liquid-impermeable film (hereinafter, called the back film) 20A positioned towards the skin of the wearer from the back nonwoven fabric 20B and formed by a water-resistive film (for example, polyethylene). The back film 20A is made from a moisture-permeable or moisture-impermeable film. The back nonwoven fabric 20B is a hydrophobic nonwoven cloth configured from an SMS (spunbond-meltblown-spunbond) nonwoven cloth, a spun bond nonwoven cloth, or a point bond nonwoven cloth. The back film 20A and the back nonwoven fabric 20B are joined with a hot-melt adhesive and the like.

As an example, the back film 20A of the exterior sheet 20 is a non-breathable film with a basis weight (mass per unit area) of 20 g/m2, and the back nonwoven fabric 20B is made from an SMS nonwoven cloth (composite nonwoven cloth of spun bond nonwoven cloth and melt-blown nonwoven cloth) with a basis weight of 13 g/m2. The hot-melt adhesive that joins the back film 20A and the back nonwoven fabric 20B is desired to be applied by spiral coating to a diameter of 15 mm at 3 g/m2.

As shown in Fig. 2, the absorber 30 is provided between the topsheet 10 and the exterior sheet 20. The absorber 30 is formed from an absorbent core 30A such as ground pulp and high absorbent polymer, and an absorbent sheet 30B, such as a tissue, for covering the absorbent core 30A.

Here, the aforementioned topsheet 10, the exterior sheet 20, and the absorber 30 are joined with each other by an adhesive (for example, hot-melt adhesive) and thermal fusion bonding.

The ventral side flaps 40A and the dorsal side flaps 40B may be a hydrophobic nonwoven cloth, a moisture-permeable or moisture-impermeable film, or a composite sheet formed by pasting together a hydrophobic nonwoven cloth and a moisture-permeable or moisture-impermeable film. A film with the main constituent as polyethylene or polypropylene, a breathable resin film, or a sheet in which a breathable resin film is joined with a nonwoven cloth such as spun bond or spun lace can be used.

As an example, the ventral side flaps 40A and the dorsal side flaps 40B can be formed from a material obtained by joining together two sheets of an SMS nonwoven cloth having a basis weight of 13 g/m2, by embossing or the hot-melt technique.

A locking unit 41 configured to lock to the front waistline region S1 is provided in each of the dorsal side flaps 40B. The locking unit 41 is provided on each of the dorsal side flaps 40B, on the outer side in the widthwise direction W on a surface on the side on which the absorber 30 is provided. The locking unit 41 is formed by a hook-and-loop fastener or an adhesive tape.

When the locking unit 41 is a hook-and-loop fastener, a female member (not shown in the figure) is provided in a region of the front waistline region S1, to which the locking unit is locked. Note that if the front waistline region S1 is configured from a nonwoven fabric, the front waistline region S1 itself can execute the role of a unit to be locked.

Gathers 50A and 50B are formed in the side regions C2 of the main body 50, respectively. The gathers 50A and 50B are formed along the longitudinal direction L of the disposable diaper 1, on the outer sides of the absorber 30 in the widthwise direction W of the disposable diaper 1. Strand-shaped members 51 are joined to gathers 50A and 50B along the longitudinal direction L of the disposable diaper 1.

In the present embodiment, the strand-shaped members 51 are made from elastic bodies having elasticity (for example, polyurethane rubber). The strand-shaped members 51 are made up of a first strand-shaped member 51A and a second strand-shaped member 51B, as well as a third strand-shaped member 51C and a fourth strand-shaped member 51D, from the inside towards the outer side in the widthwise direction W of the disposable diaper 1. The first strand-shaped member 51A and the second strand-shaped member 51B are provided between the back film 20A and the back nonwoven fabric 20B. The third strand-shaped member 51C and the fourth strand-shaped member 51D are nipped by the back nonwoven fabric 20B by folding back the back nonwoven fabric 20B which extends longer than the back film 20A in the widthwise direction W of the disposable diaper 1.

As an example, polyurethane rubber of 620 dtex can be used as the first strand-shaped member 51A and the second strand-shaped member 51B. The polyurethane rubber of 620 dtex is desired to be joined to the exterior sheet 20 by being stretched out 2.3 times. Furthermore, polyurethane rubber of 620 dtex can be used as the third strand-shaped member 51C and the fourth strand-shaped member 51D. The third strand-shaped member 51C and the fourth strand-shaped member 51D are desired to be joined to the exterior sheet 20 by being stretched out 2.5 times.

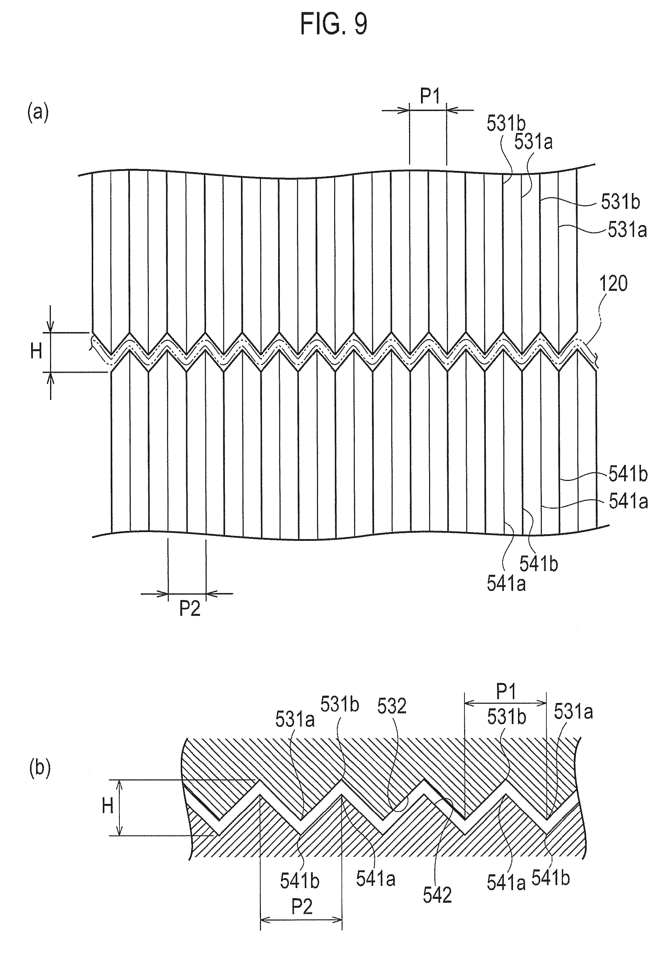

The thickness of the strand-shaped members 51 (the first strand-shaped member 51A to the fourth strand-shaped member 51D) is formed to be smaller than an interval (P1) between a plurality of tooth units 531a and an interval (P2) between a plurality of tooth units 541a of the stretching apparatus 500 (described later) which performs a stretching process on the continuous body 120 of the exterior sheet (see Fig. 9).

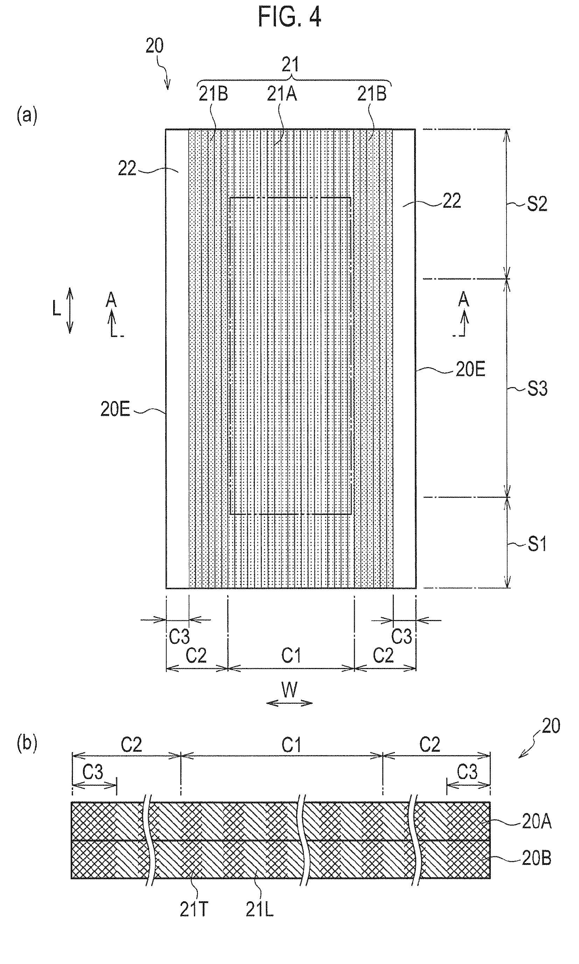

Next, the aforementioned exterior sheet 20 is explained with reference to drawings. Fig. 4 (a) is a plan view showing the exterior sheet 20 of the disposable diaper 1, and Fig. 4 (b) is an enlarged cross-sectional view (taken along the line A-A of Fig. 4(a)) of the exterior sheet 20 of the disposable diaper 1.

As shown in Fig. 4 (a), the exterior sheet 20 (the back film 20A and the back nonwoven fabric 20B) has a stretched unit 21 on which a stretching process has been performed for stretching in the widthwise direction W of the disposable diaper 1, and an unstretched unit 22 not subjected to the stretching process.

The stretched unit 21 is a region in which the exterior sheet 20 is pressed in a thickness direction of the exterior sheet 20 by an embossing roll mechanism 520 described later. The stretched unit 21 is a region that is formed with a basis weight less than the unstretched unit 22 through the stretching process performed by the embossing roll mechanism 520 described later.

The stretched unit 21 has a central stretched portion 21A positioned in the central region C1 within the crotch region S3, and side stretched portions 21B positioned in the side regions C2 within the crotch region S3. The stretched unit 21 is formed in the entire central region C1 of the disposable diaper 1 and the side regions C2 excluding edge regions C3 which include edge portions 20E positioned on the outer sides in the widthwise direction W of the exterior sheet 20.

In the stretched unit 21, a plurality of columns of unevenness are in continuity in the longitudinal direction L of the disposable diaper 1 while being formed in the widthwise direction W of the disposable diaper 1. Strictly speaking, the unevenness may not be formed in the stretched unit 21 in some cases. The unevenness also includes traces which indicate that the embossing has been performed by the stretching apparatus 500 described later.

Specifically, in the stretched unit 21, as shown in Fig. 4 (b), sparse portions 21L with a large amount of stretching and dense portions 21T whose amount of stretching is smaller than that of the sparse portions 21L are formed alternately. The sparse portions with the large amount of stretching are portions having a large ratio of width of the sheet after stretching with respect to a width of the sheet before stretching.

In the first embodiment, the edge regions C3 designate a region that a continuous body 120B of the exterior sheet, which extends longer than a continuous body 120A of backsheet in the widthwise direction W of the disposable diaper 1, is folded back on the continuous body 120B itself to form an overlap of the continuous body 120B of the exterior sheet.

It is desirable to perform the stretching process on the stretched unit 21 within a range that the stretched unit 21 does not exceed 2.5 times of the exterior sheet 20 before being subjected to the stretching process. Further, it is desirable that the stretched unit 21 has a light transmittance sufficient to allow at least the absorber 30 to be visually recognized from the outer side of the disposable diaper 1.

The unstretched unit 22 is a region that is not stretched, and is a region in which the exterior sheet 20 is not pressed in the thickness direction of the exterior sheet 20. The unstretched unit 22 is provided in the entire edge regions C3 in the longitudinal direction L of the disposable diaper 1. That is, in the side regions C2, the edge portions C3 are not subjected to the stretching process.

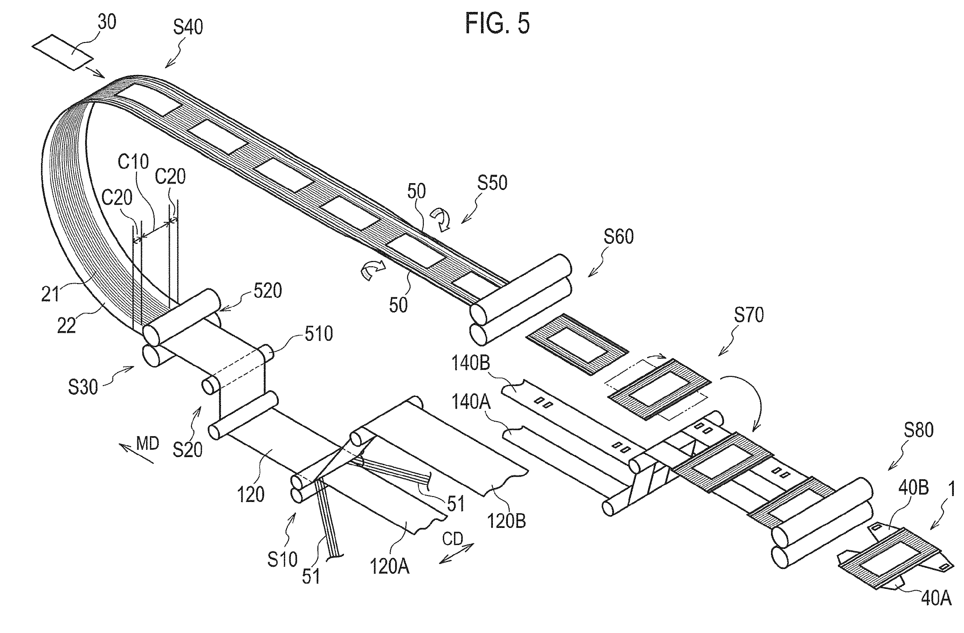

Next, a method of manufacturing the above-described disposable diaper 1 is explained with reference to drawings. Fig. 5 is a view for illustrating a method of manufacturing the disposable diaper 1 according to the first embodiment.

As shown in Fig. 5, the method of manufacturing the disposable diaper 1 includes a sheet-pasting step S10, a sheet-preheating step S20, a sheet-stretching step S30, an absorber-loading step S40, a gather-forming step S50, a sheet-cutting step S60, a sheet-rotating and transferring step S70, and a product-cutting step S80.

In the sheet-pasting step S10, the continuous body 120A of the back film 20A, and the continuous body 120B of the back nonwoven fabric 20B are pasted to each other to form the continuous body 120 of the exterior sheet 20. At this time, the strand-shaped members 51 (the first strand-shaped member 51A to the fourth strand-shaped member 51D) are joined with the continuous body 120 of the exterior sheet (between the continuous body 120A of the back film and the continuous body 120B of the back nonwoven fabric in the present embodiment), along the conveyance direction MD of the continuous body 120 of the exterior sheet. In the present embodiment, the strand-shaped members 51 are joined, in a stretched-out state, in the conveyance direction MD of the continuous body 120 of the exterior sheet.

In the sheet-preheating step S20, the continuous body 120 of the exterior sheet is heated at a predetermined temperature (for example, 100°C) by a preheating roll 510 of the stretching apparatus 500 described later.

In the sheet-stretching step S30, the continuous body 120 of the exterior sheet is stretched by the embossing roll mechanism 520 of the stretching apparatus 500 described later. Specifically, the stretching apparatus 500 stretches a region of the continuous body 120 of the exterior sheet, on which at least the absorber 30 is to be loaded, in the thickness direction of the continuous body 120 of the exterior sheet. Accordingly, the stretched unit 21 (the central stretched portion 21A and the side stretched portions 21B) is formed in a central region C10 of the continuous body 120 of the exterior sheet while the unstretched portion 22 which is not stretched is formed in side regions C20 positioned on the outer sides in the widthwise direction with respect to the central region C10. That is, the stretched unit 21 is formed in the continuous body 120 of the exterior sheet while performing conveyance in the conveyance direction MD in a state where an embossing region of the stretching apparatus 500 described later is made to correspond to the outer side with respect to a region on which the absorber is to be loaded. The embossing roll mechanism 520 configured to form the stretched unit 21 in the continuous body 120 of the exterior sheet is described in detail later.

In the absorber-loading step S40, the absorber 30 in which an absorbent core 30A is covered with an absorbent sheet 30B is loaded on the continuous body 120 of the exterior sheet on which the stretching process has been performed.

In the gather-forming step S50, by folding back the continuous body 120B of the exterior sheet which extends longer in a widthwise direction CD than the continuous body 120A of the backsheet, the third strand-shaped member 51C and the fourth strand-shaped member 51D are nipped by the continuous body 120B of the exterior sheet. In this manner, gathers 50A and 50B are formed.

In the sheet-cutting step S60, the continuous body 120 of the exterior sheet provided with the absorber 30 and the gathers 50A and 50B is cut into a size of a single product in the widthwise direction CD of the continuous body 120 of the exterior sheet.

In the sheet-rotating and transferring step S70, the cut-out exterior sheet 20 provided with the absorber 30 and the gathers 50A and 50B is rotated by 90 degrees. Further, the exterior sheet 20 rotated by 90 degrees is arranged between a continuous body 140A of the ventral side flaps on which the locking units 41 are arranged at a predetermined interval and a continuous body 140B of the dorsal side flaps.

In the product-cutting step S80, the continuous body 140A of the ventral side flaps and the continuous body 140B of the dorsal side flaps are cut into a size of a single product in the widthwise direction CD of the continuous body 140A of the ventral side flaps and the continuous body 140B of the dorsal side flaps. In this manner, the disposable diaper 1 is manufactured.

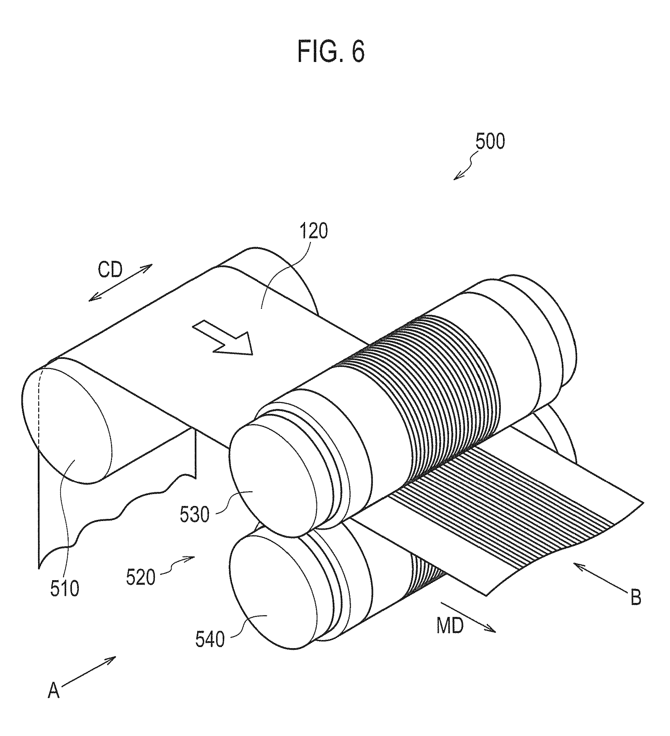

Next, the stretching apparatus 500 used in the aforementioned sheet-preheating step S20 and the sheet-stretching step S30 is explained with reference to drawings. Fig. 6 is a perspective view of the stretching apparatus 500 according to the first embodiment.

As shown in Fig. 6, the stretching apparatus 500 includes the preheating roll 510 and the embossing roll mechanism 520. The stretching apparatus 500 performs a process (called a stretching process) for stretching the continuous body 120 of the exterior sheet in the widthwise direction CD.

The preheating roll 510 is provided upstream in the conveyance direction MD of the continuous body 120 of the exterior sheet with respect to the embossing roll mechanism 520. The preheating roll 510 heats the continuous body 120 of the exterior sheet before it passes through the embossing roll mechanism 520. The preheating roll 510 has a predetermined temperature (for example, 100°C).

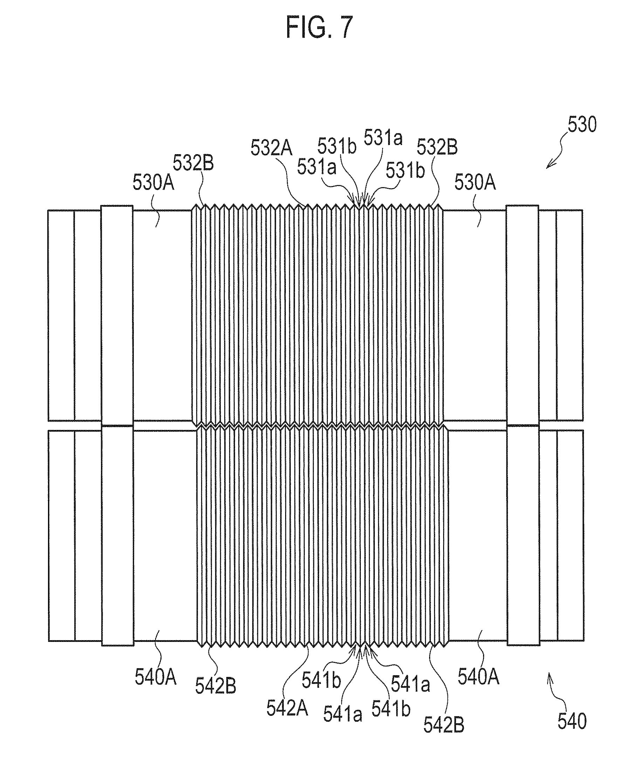

The embossing roll mechanism 520 includes a first embossing roll 530 and a second embossing roll 540. Fig. 7 is a front view (a fragmentary view taken in the direction of arrow B in Fig. 6) showing the stretching apparatus 500 according to the embodiment. As shown in Fig. 7, the central region 532A and the side regions 532B of the first embossing roll 530 have the tooth units 531a continuing in the circumferential direction of the first embossing roll 530, and untoothed units 531b formed between the tooth units 531a. A plurality of columns of the tooth units 531a and the untoothed units 531b are formed along the widthwise direction of the first embossing roll 530. Each of the tooth units 531a has a tapered shape (substantially triangular shape in the figure) from the outer circumference of the first embossing roll 530 towards the outer side, in a cross section along an axial core direction of the first embossing roll 530. In the first embossing roll 530, flat regions 530A in a flat shape in which neither tooth unit nor untooth unit is formed are formed on the outer sides in the widthwise direction with respect to the side regions 532B.

Similar to the first embossing roll 530, the central region 542A and the side regions 542B of the second embossing roll 540 have the tooth units 541a continuing in the circumferential direction of the second embossing roll 540, and untoothed units 541b formed between the tooth units 541a. Further, in the second embossing roll 540, flat regions 540A in a flat shape, in which neither tooth unit nor untooth unit is formed, are formed on the outer sides in the widthwise direction with respect to the side regions 542B. The tooth units 541a are interfitted with the untoothed units 531b of the tooth units 531a of the first embossing roll 530 while niping the continuous body 120 of the exterior sheet.

In the present embodiment, the central region 532A, the side regions 532B, the central region 542A, and the side regions 542B configure the embossing region while the flat regions 530A, 540A configure a non-embossing region.

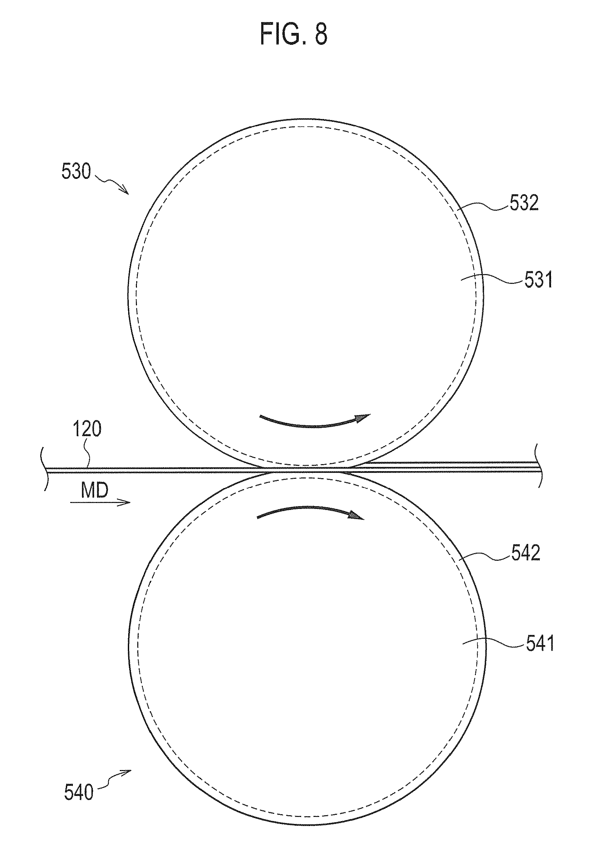

Fig. 8 is a side view (a fragmentary view taken in the direction of arrow A in Fig. 7) showing the stretching apparatus 500 according to the present invention. A length in a roll circumferential direction in which the tooth units 531a are formed corresponds to the longitudinal direction L of the disposable diaper 1.

Fig. 9(a) is an enlarged front view showing the stretching apparatus 500 according to the present embodiment. As shown in Fig. 9(a), in the embossing roll mechanism 520 having the aforementioned configuration, the tooth units 541a of the second embossing roll 540 are interfitted with the untoothed units 531b that are located between the tooth units 531a of the first embossing roll 530, via the continuous body 120 of the exterior sheet. As a result of being nipped between the tooth units 531a and the untoothed units 531b of the first embossing roll 530, and the tooth units 541a and the untoothed units 541b of the second embossing roll 540, the continuous body 120 of the exterior sheet can be stretched by as much as the unevenness (height H) of the tooth units and the untoothed units in the thickness direction of the continuous body 120 of the exterior sheet.

For example, the embossing roll mechanism 520 can perform a stretching process on the continuous body 120 of the exterior sheet for expanding the length in the widthwise direction to 2.5 times longer. The embossing roll mechanism 520 may be set to a predetermined temperature (for example, 50 to 80°C). For example, when the stretching process is performed by using an embossing roll mechanism 520 in which the diameter of the first embossing roll 530 and the second embossing roll 540 is 127 mm, the unevenness height L of the tooth units 531a and the untoothed units 531b is 1.6 mm, and each of the interval (P1) between the tooth units 531a and the interval (P2) between the tooth units 541a is 2.5 mm, and the preheating roll 510 is set to 100°C, and the embossing roll mechanism 520 is set to 80°C, a stretching process can be performed with a stretching magnification of 1.3 times on the continuous body 120 of the exterior sheet.

Generally, a material after being subjected to the stretching process becomes easily foldable along an embossing trace. However, according to the method of manufacturing the absorbent article of the first embodiment, a material of the side regions C20 of the continuous body 120 of the exterior sheet not subjected to the stretching process has a larger thickness than that of a material of the central region C10, so that a rigidity of the side regions C20 not subjected to the stretching process is greater than that of the central region C10 provided with the stretched unit 21 on which the stretching process has been performed. Therefore, as compared to a case where the stretching process is performed on the entire surface of the continuous body 120 of the exterior sheet, the turning up of edge portions and wrinkles are unlikely to occur in the exterior sheet.

Especially when, e.g., products are manufactured with a high-speed rotation, or the continuous body 120 of the exterior sheet is cut in the widthwise direction CD and the exterior sheet is rotated by 90 degrees (the sheet-rotating and transferring step S70), the continuous body 120 of the exterior sheet can also be prevented from having the turning up of the edge portions 20E (especially the turning up of the four corners of the exterior sheet 20, which is caused as a result of the cutting process).

Therefore, usage of the stretching apparatus 500 reduces more surely the possibility of defects occurring during manufacturing of the disposable diaper 1.

Further, the stretching apparatus 500 forms the stretched unit 21 in the longitudinal direction L of the disposable diaper 1, in the entire region of the central region C1, on which at least the absorber 30 is disposed. This softens the exterior sheet 20, thereby being able to manufacture a disposable diaper 1 superior in texture. Because of the softening of the exterior sheet 20, the crotch region S3 of the disposable diaper 1 easily yields, which facilitates formation of a space between the crotch of the wearer and the absorber 30. This space can catch excretions such as urine and stools, thereby preventing leakage of the excretions from the disposable diaper 1.

In the first embodiment, the embossing roll mechanism 520 performs the stretching process on the continuous body 120 of the exterior sheet with a stretching magnification of 2.5 times or less with respect to the continuous body 120 of the exterior sheet before being subjected to the stretching process. That is, the stretched unit 21 is stretched out 2.5 times or less with respect to the exterior sheet 20 before being subjected to the stretching process. Note that if the stretching magnification of the stretched unit 21 is more than 2.5 times, the exterior sheet 20 might become too thin, and damage such as breakage and perforation might occur in the exterior sheet 20.

In the first embodiment, it is desirable that the stretched unit 21 be stretched to have a thickness enough to allow the existence of the absorber 30 contained in the inside to be recognized from the outer side of the exterior sheet 20 of the disposable diaper 1. By making it possible to visually recognize the appearance of the absorber 30 from the outer side of the exterior sheet 20 of the disposable diaper 1, for example, a caretaker can determine a replacement timing (changing timing) by checking a dirt-level.

### [Second Embodiment]

Hereinafter, a stretching apparatus 500A used in a method of manufacturing an absorbent article according to a second embodiment is explained with reference to drawings. Note that the same reference numerals have been used for the same portions as the stretching apparatus described above while omitting the detailed explanation.

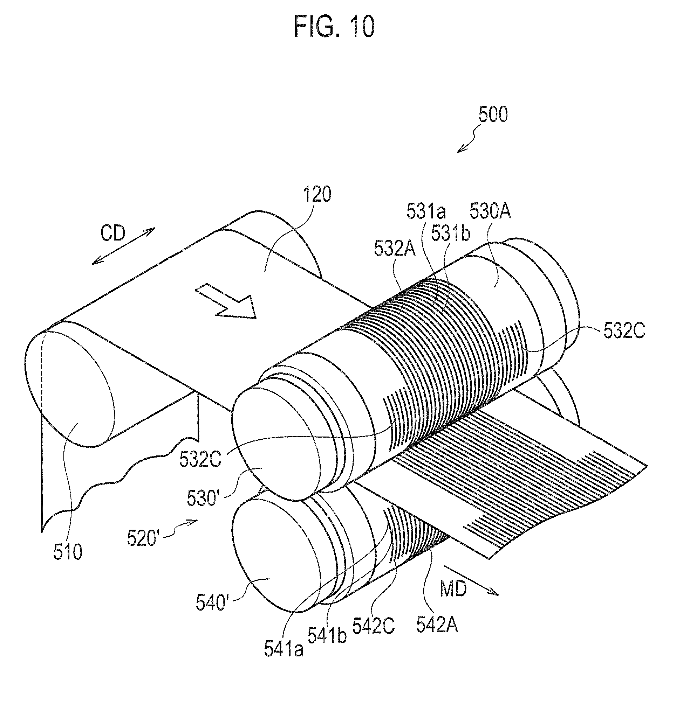

Fig. 10 is a perspective view showing the stretching apparatus 500A according to the second embodiment. The stretching apparatus 500A has an embossing roll mechanism 520'. The embossing roll mechanism 520' includes a first embossing roll 530' and a second embossing roll 540'. In the first embossing roll 530', tooth units 531a and untoothed units 531b are formed in a central region 532A and part of regions 532C on the outer side in the widthwise direction with respect to the central region 532A. In the regions 532C, the tooth units 531a and the untoothed units 532b are formed only in a partial zone in the circumferential direction of the first embossing roll 530' and the second embossing roll 540'. In the present embodiment, the regions 532C configure a partial embossing region.

In the central region 532A, a plurality of columns of the tooth units 531a and the untoothed units 531b are formed continuously in the circumferential direction of the first embossing roll 530'. In the region 532C, the tooth units 531a and the untoothed units 531b are formed in a partial zone in the circumferential direction of the first embossing roll 530'. A flat region 530A is formed on the surfaces of the first embossing roll 530' and the second embossing roll 540' except for the central region 532A and the regions 532C of the first embossing roll 530'. In the present embodiment, the flat region 530A configures a non-embossing region.

The surfaces of the first embossing roll 530' and the second embossing roll 540', except for the central region 532A and the regions 532C of the first embossing roll 530', correspond to the four corners of the exterior sheet 20 after being cut out of the continuous body 120 of the exterior sheet.

Similar to the first embossing roll 530', in the second embossing roll 540', tooth units 541a and untoothed units 541b are formed in a central region 542A and part of regions 542C on the outer side in the widthwise direction with respect to the central region 542A.

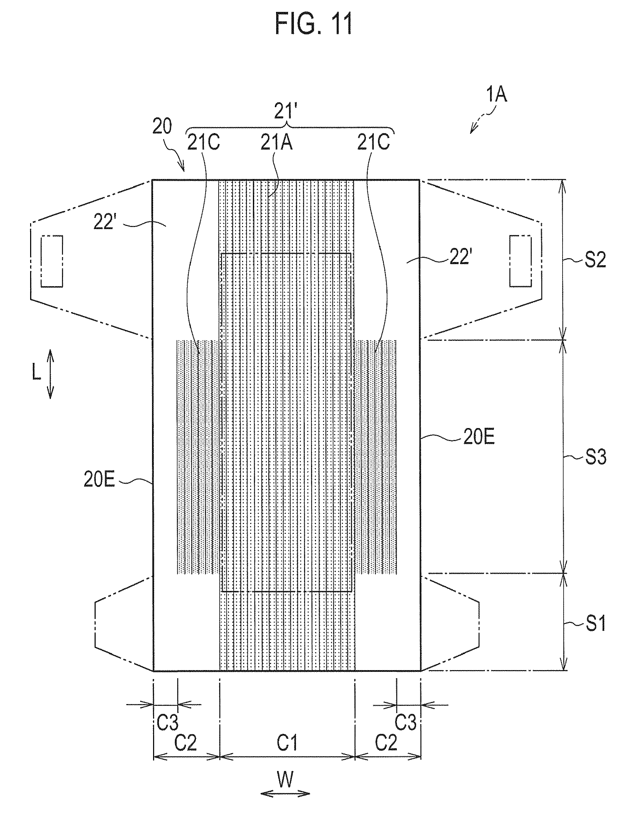

Fig. 11 is a plan view showing the exterior sheet 20 of the disposable diaper 1 on which a stretching apparatus 500A performs the stretching process. As shown in Fig. 11, the exterior sheet 20 has a stretched unit 21' on which the stretching process has been performed for stretching in the widthwise direction of the disposable diaper 1, and an unstretched unit 22' not subjected to the stretching process.

Through performance of the stretching process using the stretching apparatus 500A, a central stretched portion 21A positioned in the central region C1 within a crotch region S3 and side stretched portions 21C as part of the side regions C2 within the crotch region S3 are formed in the exterior sheet 20. In each of edge regions C3 which include edge portions 20E positioned on the outer side in the widthwise direction W of the exterior sheet 21 with respect to the side stretched portions 21C, the unstretched unit 22' on which the stretching process is not performed is formed. The unstretched unit 22' is formed by the flat region 530A of the stretching apparatus 500A.

Generally, a material after being subjected to the stretching process becomes easily foldable along an embossing trace. However, according to the method of manufacturing the absorbent article of the second embodiment, the stretching process is performed in a region corresponding to the crotch region S3 except for the edge regions C3 in the side regions C2, and in the central region C1 of the exterior sheet 20. Therefore, the material of the four corners of the exterior sheet 20 is not subjected to the stretching process and has a larger thickness than that of the material of a central region C10 (the central region C1 of the exterior sheet 20 after being cut out), thereby having a greater rigidity, so that the turning up of edge portions and winkles are less likely to occur as compared to a case where the stretching process is performed over the entire surface of the continuous body 120 of the exterior sheet. Therefore, defects can be prevented from occurring during manufacture while maintaining a configuration to ensure the flexibility in the crotch region S3 of the wearer.

### [Other Embodiment]

So far, the present invention is disclosed through the above embodiments. However, it should not be interpreted that the statements and drawings configuring a part of the present disclosure limit the present invention. From this disclosure, a variety of alternate embodiments, examples, and applicable techniques will become apparent to one skilled in the art.

For example, the embodiments of the present invention may be altered in the following ways. Specifically, the explanation for the absorbent article is based on the fact that the disposable diaper 1 is an open-type diaper, but the present invention is not limited thereto, and a pant-type diaper or a sanitary napkin can also be used.

Further, the topsheet 10, the exterior sheet 20, the absorber 30, the ventral side flaps 40A, and the dorsal side flaps 40B are not restricted to the configuration explained in the embodiment but can have other configurations and further, can be changed appropriately according to the objective.

The edge regions C3 were explained as a region in which the continuous body 120B of the exterior sheet, which extends longer than the continuous body 120A of the backsheet in the widthwise direction of the disposable diaper 1, is folded back on the continuous body 120B itself to form an overlap of the continuous body 120B of the exterior sheet. However, the edge regions C3 are not limited to the above as long as they include the edge portion 20E of the exterior sheet 20.

The stretching apparatus 500 was explained as performing the stretching process on the continuous body 120 of the exterior sheet, but is not limited thereto. The stretching apparatus 500 may perform the stretching process on a continuous body other than the continuous body 120 of the exterior sheet.

In the embodiments, the unevenness height H of the tooth units 531a and the untoothed units 531b (Fig. 9) was explained as the same between the central region 532A and the side regions 532B, but it may be different therebetween. In a case where the unevenness height of the tooth units 531a and the untoothed unit 531b in the central region 532A is higher than that of the tooth units 531a and the untoothed units 531b in the side regions 532B, a stretching magnification in the central stretched portion 21A becomes larger than that in the side stretched portions 21B. In this case, the central region C1 becomes softer than the side regions C2.

On the other hand, in a case where the unevenness height H of the tooth units 531a and the untoothed units 531b (Fig. 9) is different between the central region 532A and the side regions 532B, especially where the unevenness height H of the tooth units 531a and the untoothed units 531b in the central region 532A is smaller than that in the side regions 532B, a stretching magnification in the central stretched portion 21A becomes smaller than that in the side stretched portions 21B. In this case, the side regions C2 become softer than the central region C1.

Further, the stretching apparatus 500 was explained as an example for forming the stretched unit 21 in the exterior sheet 20, but may naturally have other configurations as long as a stretched unit 21 can be formed in the exterior sheet 20. For example, the stretching apparatus 500 need not necessarily include the preheating roll 510 and the embossing roll mechanism 520, and may be configured only from the embossing roll mechanism 520.

Furthermore, the method of manufacturing an absorbent article is not limited to the method explained in the aforementioned embodiment, and an appropriate method can be naturally selected according to the objective, as long as at least the sheet-stretching step S30 is performed.

As described above, needless to say, the present invention includes various embodiments and the like not described here. Therefore, the technical range of the present invention is to be defined only by the inventive specific matter according to the adequate claims from the above description.

The entire contents of Japanese Patent Application Laid-open No. 2010-003426 (filed on January 8, 2010) are incorporated in the present specification by reference.

### [Industrial Applicability]

According to the present invention, it is possible to provide a stretching apparatus and a method of manufacturing an absorbent article using the stretching apparatus, the stretching apparatus being capable of reducing more surely the possibility of defects occurring in a case where a continuous body of an exterior sheet or the like configuring the absorbent article is stretched in a manufacturing process of the absorbent article.

### [Reference Signs List]

1...Disposable diaper; 10...Topsheet; 20...Exterior sheet; 20A...Back film; 20B...Back nonwoven fabric; 20E...Edge portions; 21...Stretched unit; 21A...Central stretched portion; 21B...Side stretched portions; 21C...Side stretched portions; 21L... Sparse portions; 21T... Dense portions; 22...Unstretched unit; 30...Absorber; 30A... Absorbent core; 30B... Absorbent sheet; 40A...Ventral side flaps; 40B... Dorsal side flaps; 41...Locking unit; 50...Main body; 50A, 50B...Gathers; 51... Strand-shaped members; 51A... First strand-shaped member; 51B...Second strand-shaped member; 51C...Third strand-shaped member; 51D...Fourth strand-shaped member; 120...Continuous body of exterior sheet; 120A...Continuous body; 120B...Continuous body; 140A...Continuous body; 140B...Continuous body; 500...Stretching apparatus; 500A...Stretching apparatus; 510...Preheating roll; 520...Embossing roll mechanism; 530...First embossing roll; 530A...Flat region; 531a...Tooth units; 531b...Untoothed units; 532A...Central region; 532B...Side regions; 532B...Regions; 540...Second embossing roll; 540A...Flat region; 541a...Tooth units; 541b...Untoothed units; 542A...Central region; 542B...Side regions; 542C...Regions; C1...Central region; C2...Side regions; C3...Edge regions; C10...Central region; C20...Edge regions; S10...Sheet-pasting step ; S20...Sheet-preheating step; S30...Sheet-stretching step; S40...Absorber-loading step; S50...Gather-forming step; S60...Sheet-cutting step; S70...Sheet-rotating and transferring step; S80...Sheet-cutting step

## Claims

1. A stretching apparatus configured to perform a stretching process for stretching a continuous body of a material in a widthwise direction orthogonal to a conveyance direction of the continuous body of the material, the stretching apparatus comprising:
a first roll and a second roll disposed to nip the continuous body of the material, wherein:
an axial core of the first roll and an axial core of the second roll correspond to the widthwise direction of the continuous body of the material, and the first roll and the second roll have a width equal to at least a width of the continuous body of the material;
the first roll and the second roll have surfaces, each provided with:
an embossing region provided with a plurality of columns of tooth units and untoothed units, the tooth units being continuous in a circumferential direction of the first roll and the second roll, the untoothed units being formed between the tooth units; and
a non-embossing region not provided with the tooth units and the untoothed units; the embossing region is formed on each of the surfaces, in a central region on an inner side in a widthwise direction of the first roll and the second roll;
the non-embossing region is formed on the outer side in the widthwise direction of the first roll and the second roll with respect to the central region; and
the continuous body of the material is conveyed in the conveyance direction while being sandwiched between the first roll and the second roll.

2. The stretching apparatus according to claim 1, wherein:
a partial embossing region provided with the tooth units and untoothed units in only a partial zone in the circumferential direction of the first roll and the second roll, is formed on an outer side in a widthwise direction with respect to the central region of the embossing region; and
the non-embossing region is formed on each of the surfaces of the first roll and the second roll, except for the central region and the partial embossing region.

3. The stretching apparatus according to claim 1 or 2, wherein the non-embossing region corresponds to four corners of a sheet after being cut out of the continuous body of the material.

4. The stretching apparatus according to claim 1, wherein the stretching process is performed on the continuous body with a magnification of 2.5 times or less with respect to the continuous body before being subjected to the stretching process.

5. A method of manufacturing an absorbent article including a liquid-permeable topsheet, a liquid-impermeable exterior sheet, and an absorber formed between the topsheet and the exterior sheet, the method comprising:
a step of performing a stretching process to stretch a region on which the absorber is to be loaded, in a widthwise direction orthogonal to a conveyance direction while conveying a continuous body of the exterior sheet, wherein:
in the step of performing the stretching process, the continuous body of the exterior sheet is stretched by a stretching apparatus including: a first roll and a second roll disposed to nip the continuous body of the continuous body;
an axial core of the first roll and an axial core of the second roll correspond to the widthwise direction of the continuous body of the material, and the first roll and the second roll have a width equal to at least a width of the continuous body of the material;
the first roll and the second roll have surfaces, each provided with:
an embossing region provided with a plurality of columns of tooth units and untoothed units, the tooth units being continuous in a circumferential direction of the first roll and the second roll, the untoothed units being formed between the tooth units; and
a non-embossing region not provided with the tooth units and the untoothed units; the embossing region is formed on each of the surfaces, in a central region on an inner side in a widthwise direction of the first roll and the second roll;
the non-embossing region is formed on the outer side in the widthwise direction of the first roll and the second roll with respect to the central region; and
the continuous body of the exterior sheet is conveyed in the conveyance direction while being nipped between the first roll and the second roll.

6. The method of manufacturing an absorbent article according to claim 5, wherein: the continuous body of the exterior sheet is nipped between the first roll and the second roll; and
the continuous body of the exterior sheet is stretched while being conveyed in the conveyance direction in a state in which the non-embossing region of the first roll and the second roll is made to correspond to the outer side with respect to a region on which the absorber is to be disposed.
